(19) Europäisches Patentamt
European Patent Office
Office européen des brevets

(11) **EP 3 401 817 A1**

(12) **EUROPEAN PATENT APPLICATION**

(43) Date of publication:
**14.11.2018 Bulletin 2018/46**

(51) Int Cl.:
***G06F 19/00*** *(2018.01)*

(21) Application number: **17170699.7**

(22) Date of filing: **11.05.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **SU, Jing (Shelly)**
**5656 AE Eindhoven (NL)**
• **KELLY, Declan Patrick**
**5656 AE Eindhoven (NL)**
• **RONDA, Cornelis Reinder**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **NOCTURNAL ASTHMA MONITORING**

(57) A sleep monitoring system (10) for detecting asthma symptoms during the sleep of a subject is disclosed. The system comprises a $CO_2$ sensor (21) and a processor (31) communicatively coupled to the $CO_2$ sensor, wherein the processor is adapted to monitor a change in a $CO_2$ concentration in a space in which the subject is asleep from sensor data produced by the $CO_2$ sensor over a monitoring period; compare the monitored change of the $CO_2$ concentration in said space against a benchmark for said subject; and identify an asthma symptom exhibited by said subject if the monitored change of the $CO_2$ concentration in said space exceeds the benchmark for said subject for at least part of said monitoring period. A method of detecting asthma symptoms during the sleep of a monitored subject and a computer program product for implementing such a method on a sleep monitoring system are also disclosed.

FIG. 1

**Description**

FIELD OF THE INVENTION

The present invention relates to a sleep monitoring system and method for detecting asthma symptoms during the sleep of a subject, as well as to a computer program product for implementing such a method.

BACKGROUND OF THE INVENTION

Sleep is a critical part of our lives. It ensures that our bodies rest and can repair and is therefore crucial for long-term health. It is therefore important that people, i.e. subjects, sleep properly. Without proper sleep, chronic health issues may arise. For this reason, many solutions have been proposed that facilitate the monitoring of sleep by a subject, for example to diagnose sleep disorders.

An example of such a sleep disorder is disrupted sleep though the onset of asthma symptoms including wheezing, shortness of breath and coughing, which may be caused by bronchospasms and variable degrees of inflammation of the airways of the lungs. Such nocturnal asthma symptoms have been associated with increased mortality and decreased quality of life, for example due to the poor quality of sleep experienced by the patient.

In order to manage such a condition in adults and children, it is important to obtain reliable clinically relevant data that can be used by the patient or a clinician to establish whether the condition is under control. To this end, the patient may be presented with a questionnaire such as the asthma control test (ACT), which includes questions about night time sleep disruption due to the asthma over a period of time, typically four weeks.

However, it is rather difficult for the patient to correctly assess the severity of the asthma symptoms during sleep, because some of the symptoms may reduce the quality of sleep whilst not leading to the patient waking up or may lead to such a short disruption of the sleep that the patient will not remember it the next morning, such that reliance on the patient's own monitoring of his or her symptoms typically leads to underreporting of the symptoms, thereby making it difficult to decide on the appropriate treatment regime of the patient in order to keep the condition under control. Although this is problematic in adults, it is particularly cumbersome for child patients, who often are incapable of providing the clinically relevant information on which a reliable assessment of the condition can be based. Therefore, there exists a need for monitoring devices that can assist in determining the onset of symptoms potentially caused by asthma during the sleep of a subject (patient).

US 2016/0249838 A1 discloses a system and method to detect a possible respiratory blockage by using personalized carbon dioxide ($CO_2$) concentration change patterns to prevent deaths by respiratory blockage such as by choking or asthma. Blood $CO_2$ levels are monitored through a skin-mount patch including a non-dispersive infrared sensor and a warning signal is generated if these levels become abnormally high. However, this solution is considered non-ideal for a number of reasons. Firstly, some patients may find it uncomfortable to wear such a patch whilst sleeping, as they find that the sensation of having to wear the patch disrupts their sleep. Secondly, this prior art system is not adapted to provide an overall quality of sleep indication as a function of the onset of asthma symptoms during the sleeping period, as the system is incapable of determining whether the patient is awake or asleep.

SUMMARY OF THE INVENTION

The present invention seeks to provide a sleep monitoring system that is capable of monitoring the effects of asthma symptoms during the sleep of a patient being monitored in an unobtrusive manner.

The present invention further seeks to provide a method of detecting asthma symptoms during the sleep of the subject being monitored in an unobtrusive manner.

The present invention further seeks to provide a computer program product for implementing such a method on a sleep monitoring system.

According to an aspect, there is provided a sleep monitoring system for detecting asthma symptoms during the sleep of a subject, the system comprising a $CO_2$ sensor and a processor communicatively coupled to the $CO_2$ sensor, wherein the processor is adapted to monitor a change in a $CO_2$ concentration in a space in which the subject is asleep from sensor data produced by the $CO_2$ sensor over a monitoring period; compare the monitored change of the $CO_2$ concentration in said space against a benchmark for said subject; and identify an asthma symptom exhibited by said subject if the monitored change of the $CO_2$ concentration in said space exceeds the benchmark for said subject for at least part of said monitoring period.

The present invention is based on the insight that the amount of $CO_2$ expelled by a subject, i.e. a person, which is a function of the state of activity of that subject, can not only be used to accurately determine whether the subject is awake or asleep, but can further distinguish between normal sleep, i.e. sleep in which the subject does not suffer from asthma symptoms, and abnormal sleep, i.e. sleep in which such asthma symptoms compromise the sleep efficiency of the subject. Consequently, by monitoring $CO_2$ levels with the sensor and processing the monitored $CO_2$ levels with the processor, asthma symptom information can be derived during the subject's sleep without having to contact the subject being monitored, thereby providing clinical data with improved accuracy compared to self-reporting of asthma symptoms by the subject (patient) such that the subject's asthma can be controlled more accurately based on the provided asthma symptom information.

The sleep monitoring system preferably further comprises a monitoring result reporting device communicatively coupled to the processor and adapted to generate a report of said monitoring period, said report including an indication of identified asthma symptoms in order to report the monitoring result to a user of the system. However, it is noted for the avoidance of doubt that such a monitoring result reporting device may be omitted, for example in a scenario where the sleep monitoring system provides those results to a remote device for interpretation, e.g. over a network such as the Internet in which case the monitoring results may be remotely interpreted, such as by a trained professional. In an embodiment, the processor further is adapted to sample the sensor data at a defined sampling frequency of at least 0.1 Hz. By sampling at least every 10 seconds, changes in $CO_2$ concentration indicative of an asthma symptom occurring over a short period of time, e.g. concentration changes caused by coughing or shortness of breath, can be monitored by the sleep monitoring system, thereby improving its ability to produce results of diagnostic relevance. For example, the processor may be adapted to identify an asthma symptom in the form of a cough of the subject if a monitored change in said $CO_2$ concentration between at least two contiguous sensor data samples exceeds the benchmark by at least a defined amount.

The sleep monitoring system may further comprise a counter adapted to count the number of identified asthma symptoms during the monitoring period. For example, the counter may count of the number of detected coughs during the sleep monitoring period, which may provide clinically relevant information about the severity of the asthma symptoms the subject being monitored suffers from.

In a preferred embodiment, the sleep monitoring system is operable in a calibration mode in which the benchmark for the subject is obtained. This further improves the accuracy of the sleep monitoring system compared to systems in which predefined benchmarks, e.g. benchmarks based on at least one of age, gender, weight and size, are being utilized.

In an embodiment, the monitoring period is defined by a first time period initiated by a first indication that the subject is attempting to sleep and terminated by a second indication that the subject is getting up; and wherein the processor is adapted to determine a sleep efficiency of the subject during the monitoring period by identifying an awake time period of the subject when a rate of increase in the monitored $CO_2$ concentration is greater than a first threshold; identifying a light sleep time period of the subject when a rate of increase in the monitored $CO_2$ concentration is between the first threshold and a second threshold; and identifying a deep sleep phase of the subject when a rate of increase in the monitored $CO_2$ concentration is below the second threshold. This for example facilitates evaluation of the sleep efficiency of the subject being monitored, as well as the impact of the asthma symptoms on this sleep efficiency.

To this end, the processor may be further adapted to determine a cause for the subject being awake by evaluating a rate of increase in the monitored $CO_2$ concentration during a time period immediately preceding the awake time period. In this manner, the sleep monitoring system can distinguish between 'normal' waking up and waking up caused by discomfort associated with the onset of asthma symptoms, which information may be utilized in determining the sleep efficiency of the monitored subject. In particular, the processor may be adapted to differentiate between causes for waking up including normal waking up and asthma symptoms including coughing, shortness of breath and wheezing.

Optionally, the sleep monitoring system further comprises at least one further sensor communicatively coupled to the processor, wherein the processor is adapted to identify the asthma symptoms based on respective sensor data from the $CO_2$ sensor and the at least one further sensor. This for instance is beneficial where the sleep monitoring system is configured to determine a sleep efficiency metric for the subject, where the sensor data provided by the further sensor may be used to determine the point in time at which the subject begins the attempt to fall asleep or to determine the point in time at which the subject wakes up. For example, the further sensor may be a light sensor, a sound sensor or a user interface sensor.

In a further embodiment, the sleep monitoring system further comprises an asthma medication release device responsive to the processor. This has the advantage that asthma medication may be automatically released in the proximity of the subject being monitored upon detection of asthma symptoms, thereby improving the management of the condition and reducing the risk of serious complications developing from the onset of these symptoms.

The sleep monitoring system may be provided as a single unit, stand-alone apparatus or the sleep monitoring system may be at least partially comprised by an air conditioning apparatus, an air purification apparatus, a respirator apparatus or a humidification apparatus. The sleep monitoring system alternatively or additionally may be realized by a distributed architecture in which the sleep monitoring system comprises a first device comprising the $CO_2$ sensor and a second device comprising the processor, the first device and the second device each comprising a wireless communication module for establishing a wireless communication link between the first device and the second device, wherein the second device is a wearable device or a mobile communication device.

According to a further aspect, there is provided a method of detecting asthma symptoms during the sleep of a subject, the method comprising periodically receiving sensor data indicative of a $CO_2$ concentration in a space in which the subject is asleep during a monitoring period; monitoring the $CO_2$ concentration over said monitoring period; comparing a change in the monitored $CO_2$ concentration in said space against a benchmark for said subject; and identifying an asthma symptom exhibited by said subject if the monitored change of the $CO_2$ concentration in said space exceeds the benchmark for said subject for at least part of said monitoring period. Such a method facilitates the monitoring of asthma during the sleep of a subject such as a child (or adult) without having to physically contact the subject, thereby providing an unobtrusive manner of obtaining accurate data regarding the onset of asthma symptoms during sleep of the subject being monitored.

The method preferably further comprises determining a sleep efficiency of the subject during the monitoring period by identifying an awake time period of the subject when a rate of increase in the monitored $CO_2$ concentration is greater than a first threshold; identifying a light sleep time period of the subject when a rate of increase in the monitored $CO_2$ concentration is between the first threshold and a second threshold; and identifying a deep sleep phase of the subject when a rate of increase in the monitored $CO_2$ concentration is below the second threshold; the determining of said sleep efficiency further comprising determining a cause for the subject being awake by evaluating a rate of increase in the monitored $CO_2$ concentration during a time period immediately preceding the awake time period from a set of causes including normal waking up and asthma symptoms including coughing, shortness of breath and wheezing, such that the overall effect of the asthma symptoms on the quality of sleep of the monitored subject can be determined.

According to yet another aspect, there is provided a computer program product comprising a computer readable storage medium having computer readable program instructions embodied therewith for, when executed on a processor of a sleep monitoring system of any of the herein described embodiments, cause the processor to implement the method according to embodiments of the present invention.

BRIEF DESCRIPTION OF THE DRAWINGS

Embodiments of the invention are described in more detail and by way of non-limiting examples with reference to the accompanying drawings, wherein:

FIG. 1 schematically depicts a sleep monitoring system according to an embodiment;
FIG. 2 is a graph depicting typical human ventilation volumes associated with different states of awareness;
FIG. 3 is a graph depicting measured indoor $CO_2$ levels for a room in which a person is active;
FIG. 4 is a graph depicting measured indoor $CO_2$ levels for a room in which a person is awake but resting;
FIG. 5 is a graph depicting measured indoor $CO_2$ levels for a room in which a person is asleep;
FIG. 6 is a block diagram schematically depicting the various sleep phases during a typical sleep cycle of a person;
FIG. 7 is a graph depicting the effects of a subject's coughing on $CO_2$ emissions in a location within a space proximal to the subject;
FIG. 8 is a flowchart of a method according to an example embodiment;
FIG. 9 is a flowchart of an aspect of the method of FIG. 8 in more detail;
FIG. 10 schematically depicts a sleep monitoring system according to another embodiment; and
FIG. 11 schematically depicts a sleep monitoring system according to yet another embodiment.

DETAILED DESCRIPTION OF THE EMBODIMENTS

It should be understood that the Figures are merely schematic and are not drawn to scale. It should also be understood that the same reference numerals are used throughout the Figures to indicate the same or similar parts.

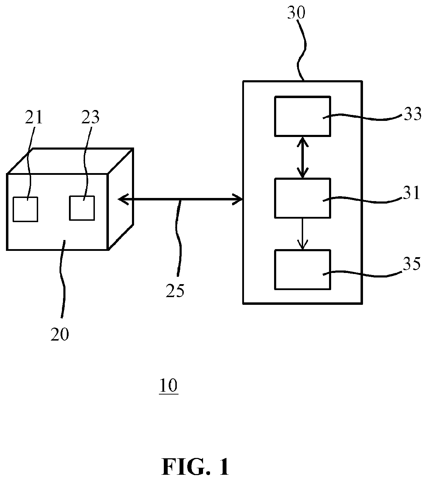

FIG. 1 schematically depicts a sleep monitoring system 10 according to an embodiment. The sleep monitoring system 10 is adapted to monitor the sleep of a subject in a confined space such as a bedroom in which a sensor device 20 of the sleep monitoring system 10 is positioned. The sensor device 20 at least comprises a $CO_2$ sensor 21 and may comprise one or more further sensors 23, which may include a light sensor, a sound sensor, e.g. a microphone, a user input sensor, e.g. a user interface, and so on.

The sensor device 20 maybe a stand-alone device, e.g. a sensor box or the like that may be positioned in close vicinity to the subject to be monitored. For example, the sensor device 20 maybe dimensioned such that it can be clipped or otherwise secured to a bed, e.g. to a headboard of the bed, in which the subject sleeps, such that a change in the $CO_2$ concentration caused by exhalation of $CO_2$ by the subject can be accurately monitored with the sensor device 20 before the $CO_2$ diffuses into the total volume of air within the confined space in which the sensor device 20 is positioned, e.g. a bedroom in which the subject sleeps. The sensor device 20, or at least the $CO_2$ sensor 21, should be positioned such that accurate monitoring of fluctuations in $CO_2$ levels local to the subject being monitored as caused by the subject's breathing is facilitated. For example, the $CO_2$ sensor 21 maybe positioned within 30 cm from the subject's mouth and nose although other suitable distances may be possible, e.g. depending on the ventilation conditions within the room in which the subject is located.

In alternative embodiments, the sensor device 20 may form part of an apparatus adapted to alter the condition of the atmosphere within the confined space as will be explained in more detail below. For example, such an apparatus may be adapted to adjust at least one of the purity, humidity, temperature and scent level in the atmosphere (air) in the confined space. Such functionality for example may be included in an air purification apparatus, an air conditioning apparatus, an air humidification apparatus, a scent release apparatus or any apparatus that includes one or more of the above functionality.

The sleep monitoring system 10 typically comprises a computing device 30 including a processor 31. As shown in FIG. 1, the computing device 30 may be a separate device to the sensor device 20. For example, the computing device 30 may be any suitable computing device, such as a personal computer, e.g. a desktop computer or a laptop computer, a tablet computer, a personal digital assistant, a mobile communication device such as a smartphone, a wearable smart device such as a smart watch, and so on. The computing device 30 may form an assembly with the sensor device 20. In such an assembly, the computing device 30 may be a discrete entity or may form part of an apparatus adapted to alter the condition of the atmosphere within the confined space, i.e. such an apparatus may comprise the processor 31. The processor 31 may be any suitable processor, e.g. a generic processor or an application-specific processor. The computing device 30 may further comprise a data storage device 33 communicatively coupled to the processor 31.

The computing device 30 is arranged to communicate with the sensor device 20 to obtain $CO_2$ levels in the confined space in which the subject is located as determined with the $CO_2$ sensor 21. The $CO_2$ sensor 21 and the further sensor(s) 23 if present are communicatively coupled to the computing device 30 over a communication link 25 such that the processor 31 can receive sensor readings from such sensors. Such a communication link may be a wired communication link, e.g. in case the sensors 21, 23 are integral to the computing device 30, or maybe a wireless communication link, e.g. in case the sensors 21, 23 are located in a different device to the computing device 30, e.g. in a separate sensor device 20. To this end, the respective devices communicatively coupled over such a wireless communication link may include a wireless transceiver (not shown). The devices may communicate with each other through their respective wireless transceivers using any suitable wireless communication protocol, e.g. Bluetooth, Wi-Fi, a mobile communication protocol such as 2G, 3G, 4G or 5G, a suitable near-field communication (NFC) protocol or a proprietary protocol. In case of such wireless communication, the respective devices may communicate directly with each other or may communicate with each other through an intermediary such as a wireless bridge, a router, a hub, and so on. Any suitable embodiment of wired or wireless communication between such respective devices may be contemplated.

The processor 31 may be further communicatively coupled to a data storage device 33, here shown to form part of the computing device 30. Such a data storage device may be any suitable device for storing digital data, e.g. a random access memory, a cache memory, a Flash memory, a solid state storage device, a magnetic storage device such as hard disk, an optical storage device and so on. Alternatively, the data storage device 33 may be separate from the computing device 30, e.g. a network storage device or a cloud storage device accessible to the processor 31 over a network such as a LAN or the Internet. The processor 31 may store sensor data received from the connected sensors 21, 23 in the data storage device in order to collect and store historical sleep information obtained for the subject in the confined space, for example to analyze the sleep efficiency of that subject as will be explained in more detail below.

In FIG. 1, the computing device 30 further comprises a monitoring result reporting device 35 under control of the processor 31. Such a device may be any device that capable of producing an output that can be detected by one of the human senses. For example, the device 35 may be adapted to produce a visible or audible output. The processor 31 may be adapted to generate a control signal indicative of a determined sleep efficiency of the subject with the processor 31, which control signal triggers the device 35 to produce a sensory output indicating the determined sleep efficiency. For example, the monitoring result reporting device 35 may comprise a display adapted to display the determined sleep efficiency (or sleep efficiency history) of the subject, and/or any data pertaining to monitored asthma symptoms during the sleep of the subject being monitored as will be explained in further detail below.

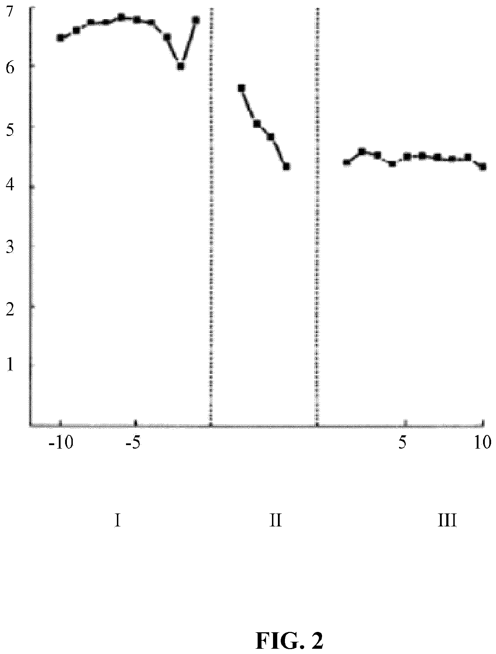

FIG. 2 provides proof of concept of the ability to detect different states of awareness, i.e. a distinction between a subject being awake or asleep. FIG. 2 depicts a graph in which three sleep phases are identified. Phase I is awake, phase II is a transition to a state of sleep and phase III is a state of sleep, with the X-axis displaying time (in minutes) and the Y-axis displaying ventilation of the subject (in l/min). This graph therefore clearly depicts a distinct decrease in ventilation (breathing) volumes upon the subject going from a state of being awake to a state of being asleep. Consequently, the amount of $CO_2$ expelled going from a state of being awake to a state of being asleep is therefore also reduced. The monitored amount of $CO_2$ expelled by a subject under monitoring during a unit period of time can be used as an indicator of whether the subject is awake or asleep. For example, if the amount of $CO_2$ expelled during such a unit period of time exceeds a defined threshold, this may be considered indicative of the subject being awake, whereas if the amount of $CO_2$ expelled during such a unit period of time falls below this defined threshold, this may be considered indicative of the subject being asleep.

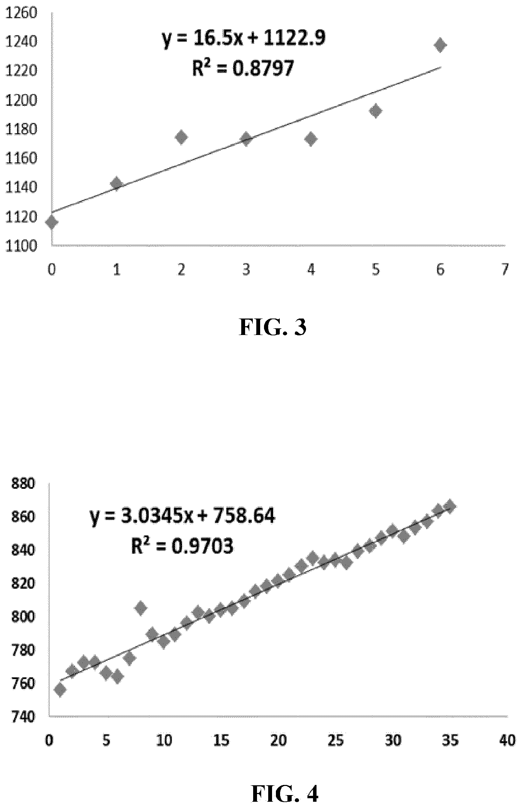

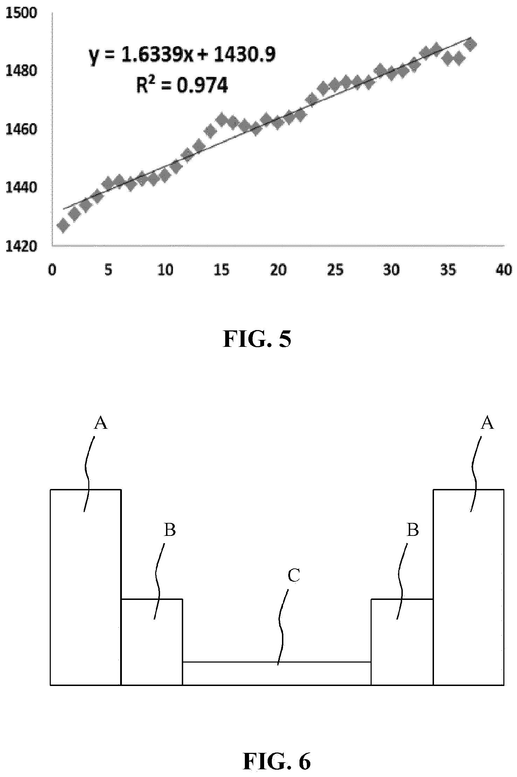

The feasibility of using the monitoring of $CO_2$ levels to monitor the sleep of a subject is further demonstrated by FIG. 3-5, in which the levels of $CO_2$ expelled by a subject during exercise (FIG. 3), rest (FIG. 4) and sleep (FIG. 5) were monitored with a $CO_2$ sensor over a period of time within the same confined space (i.e. a space having a constant volume of 29.25 m³), with hermetically sealed windows and doors to minimize the loss of $CO_2$ from the confined space. During exercise, the

monitored $CO_2$ levels translated into a rate of $CO_2$ increase of 16.5 ppm/min. In a rest state (i.e. the subject being awake but resting), the monitored $CO_2$ levels translated into a rate of $CO_2$ increase of 3.0 ppm/min, whereas in a sleep state of the subject, the monitored $CO_2$ levels translated into a rate of $CO_2$ increase of 1.6 ppm/min.

As will be immediately understood, the absolute values of these rates of $CO_2$ increase are dependent of several factors such as volume of the confined space, bodyweight and/or lung capacity of the monitored subject, rate of loss of $CO_2$ from the confined space, and so on. However, the data in FIG. 3-5 clearly demonstrates that for a particular subject, a clear difference exists in the rate at which $CO_2$ levels rise in the confined space between the various physical states of the monitored subject. Consequently, it is clearly demonstrated that by determining the rate of increase of the $CO_2$ level and comparing this rate against a defined threshold, a determination can be made about the physical state of the monitored subject, e.g. whether the subject is awake or asleep.

Moreover, it is well-known per se that a person in a light state of sleep produces a higher volume of ventilation (breathing) per unit time compared to a person in a deep state of sleep, such that a distinction between a light sleep and a deep sleep of a monitored subject may also be made by monitoring a rate of increase of $CO_2$ levels in the confined space and comparing the determined rate of increase of $CO_2$ levels in the confined space against a further defined threshold, with a light sleep being detected when the determined rate of increase of $CO_2$ levels in the confined space is above the further defined threshold and a deep sleep being detected when the determined rate of increase of $CO_2$ levels in the confined space is below the further defined threshold.

In an embodiment, the sleep monitoring system 10 maybe configured to determine a particular physical state of the monitored subject in accordance with Table 1 (threshold 1 being higher than threshold 2):

Table 1

| State | Threshold 1 | Threshold 2 |
|---|---|---|
| Awake | Above | Above |
| Light sleep | Below | Above |
| Deep Sleep | Below | Below |

As previously mentioned, the absolute values of threshold 1 and threshold 2 will depend from a number of factors, such as such as volume of the confined space, bodyweight and/or lung capacity of the monitored subject, rate of loss of $CO_2$ from the confined space, and so on. In an embodiment, the respective thresholds to be applied by the sleep monitoring system 10 may be obtained through calibration of the system. This may be achieved in any suitable manner. For example, at least the sensor device 20 of the sleep monitoring system 10 may be placed within the confined space and used to monitor the subject over a period of time, e.g. during a night, in which the subject sleeps within the confined space. The data collected with the sensor device 20 may be evaluated to identify typical changes in the rate of increase of $CO_2$ levels within the confined space, which typical changes will be indicative of a change in physical state of the subject, e.g. a transition from a state of being awake to a state of light sleep or a transition from a state of light sleep to a state of deep sleep. Consequently, the various physical states can be readily identified in the collected data, such that the applicable values of Threshold 1 and Threshold 2 associated with (transitions between) these various physical states can be readily derived from the collected data. In order to improve the accuracy of the thus extracted thresholds, the data collection during calibration may be repeated a number of times, e.g. over a number of nights. The sleep monitoring system 10 may have a calibration mode that can be user-activated. For example, the sleep monitoring system 10 may comprise a user interface, e.g. on the sensor device 20 or the computing device 30 that allows the user to activate the calibration mode, e.g. after installation of the sensor device 20 in the vicinity of the location in which the subject to be monitored intends to sleep.

In an embodiment, the sleep monitoring system 20 is adapted to determine the sleep efficiency of the subject being monitored. The sleep efficiency SE may be defined as follows:

$$SE = \frac{\Delta T_{sleep}}{\Delta T_{total}}$$

$\Delta T_{total}$ is the total time the subject is attempting to sleep, whereas $\Delta T_{sleep}$ is the total time the subject actually is asleep. $\Delta T_{total}$ may be defined as a first time period initiated by an indication that the subject is attempting to sleep and terminated by an indication that the subject is getting up. The indication that the subject is getting up typically follows an indication that the subject has been asleep although this is not strictly necessary; for example in a scenario where the subject did not manage to sleep at all, such an indication of the subject being asleep would not be obtained.

The total time $\Delta T_{total}$ maybe determined in a number of ways. For example, the start point of this period may be determined by collecting an indication with a further sensor 23 that the subject is attempting to sleep. This for example may be a pressure sensor for detecting the subject entering the bed, which pressure sensor for instance may be attached to a pillow or mattress or the like. However, such an indicator may be less accurate if the subject initially engages in relaxing activities before attempting to sleep, such as reading or watching TV. Alternatively, the further sensor 23 maybe a light sensor that detects a change in light level in the confined space. In this manner, if the subject switches off a light within the confined space such as a bedside lamp or the TV, this may be interpreted as an indication of the subject attempting to go to sleep, and such an indication may be an accurate indication from which the determination of the time period $\Delta T_{total}$ may be initiated. Similarly, a sound sensor such as a microphone may be used for this purpose, as the user switching off the TV or stopping reading may be detected by a reduction in noise levels within the confined space. In yet another embodiment, the subject may provide a user input on a user input sensor 23 of the sensor device 20, e.g. on the user interface, to provide a particularly accurate indication of the subject initiating attempting to sleep. The endpoint of the time period $\Delta T_{total}$ may be determined in a similar manner, for example by detecting an alarm going off, by the subject switching on a light, from an increase in the rate at which $CO_2$ is expelled by the subject is determined with the $CO_2$ sensor 21, and so on.

$\Delta T_{sleep}$ may be defined as a second time period initiated by an indication that the subject is asleep and terminated by an indication that the subject is awake that follows the indication that the subject is asleep. In case of a disrupted sleep pattern, the subject may experience a number of periods during which the subject is asleep. In such a scenario, the total period $\Delta T_{sleep}$ that the subject was asleep may be obtained by summing all periods during which it was determined that the subject was asleep.

As will be understood from the foregoing, the total time $\Delta T_{sleep}$ may be determined using the $CO_2$ sensor data collected with the sensor device 20. For example, the sensor device 20 may periodically sample the $CO_2$ levels in the confined space in which the subject is attempting to sleep, which periodic data may be used to determine the total time $\Delta T_{sleep}$. For example, the total time $\Delta T_{sleep}$ may be determined by counting the number of data points in the periodic data for which the rate of increase of the $CO_2$ level relative to the previously captured data point was below Threshold 1. Other suitable ways of determining $\Delta T_{sleep}$ from the collected sensor data will be immediately apparent to the skilled person.

The sleep monitoring system 10 may be further refined, for example to factor in scenarios in which the monitored subject temporarily leaves the bed, e.g. for a toilet break or the like. To this end, the sleep monitoring system 10 for example may be configured to continue determining the time period $\Delta T_{total}$ if it is determined that the subject returns to bed within a defined period of time. This may be determined in any suitable manner, e.g. using sensor data provided by the $CO_2$ sensor 21 and/or one or more of the further sensors 23 as previously explained. Other refinement approaches will be apparent to the skilled person.

In an embodiment, the sleep monitoring system 10 is further adapted to calculate the sleep onset latency (SOL) for the monitored subject. The sleep onset latency may be defined as the time period between the point in time at which the subject attempts to go to sleep and the point in time at which the subject actually falls asleep. The point in time at which the subject attempts to go to sleep and the maybe determined the point in time at which the subject actually falls asleep may be determined as previously explained.

In an embodiment, the sleep monitoring system 10 may be adapted to provide an indication of the calculated sleep efficiency SE, optionally including an indication of the sleep onset latency SOL, on the sensory output device 35 such that the monitored subject may be made aware of his or her sleep efficiency. To this end, the sensory output device 35 may be included in a computing device 30 that is portable, e.g. a tablet device or mobile communications device such as a smart phone, or wearable device, e.g. a smart watch or the like that may be worn by the monitored subject during sleep. This has the further advantage that if the sensor device 20 is separate to the computing device 30, a short range wireless communication between the sensor device 20 and the computing device 30 maybe deployed, e.g. NFC or Bluetooth, which may be beneficial in terms of energy efficiency.

The sleep monitoring system 10 may be adapted to build a history of sleep efficiencies to allow evaluation of the sleep history of the subject to be monitored. For example, the processor 31 may be adapted to store sleep monitoring data and/or a sleep efficiency calculated from the sleep monitoring data in the data storage device 33. The sleep monitoring system 10 may comprise a display as the sensory output device 35 on which the sleep history stored in the data storage device 33 may be displayed. In this manner, a history of the sleep efficiency of the monitored subject may be displayed and evaluated, which may provide valuable insights into typical sleep behaviors of the monitored subject. Such insights for instance may be used to determine if certain physical symptoms of the monitored subject may be explained by the sleep efficiency of the monitored subject over a period of time.

In accordance with the present invention, the sleep monitoring system 10 is further configured to determine asthma symptoms during the sleep period of the subject being monitored. Such asthma symptoms broadly can be categorized in three different categories; coughing, shortness of breath and wheezing, each of which can be categorized by abnormal and distinguishable increases in $CO_2$ emissions by the subject being monitored by the system 10 as will be explained in more detail below.

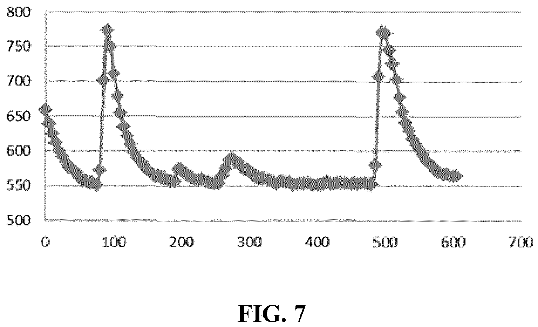

FIG. 7 is a graph depicting the monitoring of $CO_2$ levels (y-axis, in PPM) within a confined space (here an office) over time (x-axis, in seconds) in which a subject is present. The spikes at t ~ 110s and t ~ 510s are characteristic of a sudden increase in CO2 levels within the monitored space caused by the subject coughing. In order for the sleep monitoring system 10 to be able to monitor such coughing events, the processor 31 preferably is configured to sample the sensor data are provided by the $CO_2$ sensor 21 at least every 10 seconds, i.e. at a sample rate of at least 0.1 Hz, such that such transient

sharp increases in $CO_2$ levels within the monitored space can be accurately detected by the sleep monitoring system 10. For example, the processor 31 may be configured to determine that an increase in $CO_2$ levels is to be interpreted as a coughing event when the monitored change in the $CO_2$ concentration between at least two contiguous sensor data samples exceeds a benchmark by at least a defined amount. The sleep monitoring system 10 may further comprise a counter (not shown) in which the detected asthma symptoms, e.g. the number of coughing events, during the sleep of the subject being monitored are counted. This count for example may be used to quantify the severity of an asthma attack during the sleeping period of the monitored subject, and/or may be used in the determination of the sleep efficiency of the monitored subject as will be explained in further detail below.

The aforementioned benchmark utilized by the processor 31 typically is a personalized benchmark for the said subject. The personalisation of the benchmark may be achieved in some embodiments by a user of the sleep monitoring device 10 selecting a particular benchmark from a plurality of pre-stored benchmarks through a user interface of the sleep monitoring system 10, for example by specifying one or more identifiers of the subject to be monitored such as age, gender, weight, height, lung capacity, and so on, based on which an appropriate pre-stored benchmark may be selected by the processor 31 for use in the monitoring of the subject. In a preferred alternative embodiment, the benchmark may be obtained in a calibration mode of the sleep monitoring system 10 as previously explained. Such a benchmark for example may define changes in $CO_2$ concentration over time within the space in which the target subject is being monitored caused by the subject exhibiting normal breathing behaviour.

In an embodiment, the benchmark may define the expectation value of changes in $CO_2$ concentration over time when the target subject is breathing normally whilst being awake, as the changes in the $CO_2$ concentration over time as caused by the onset of asthma symptoms typically causes the amounts of $CO_2$ emitted by the monitored subject to exceed the amounts of $CO_2$ emitted by the subject during normal breathing whilst awake. For example, asthma sufferers tend to brief through their mouths rather than their noses when suffering from an asthma attack, which typically is accompanied by an increased rate of breathing due to the fact that the breathing is more shallow compared to normal breathing. It is well-known per se in medical science that asthma sufferers suffer from chronic hyperventilation; on average, asthma sufferers breathe 12 to 14 L of air per minute compared to 4-6 L of air per minute during normal breathing. This is typically accompanied by an increased rate of breathing, e.g. about 25 breaths per minute for an asthma sufferer.

It has been found that above Table 1 may be extended by the application of a further threshold, e.g.

the personalized benchmark as mentioned above, which typically is a higher threshold than Threshold 1 in Table 1 as the rate of $CO_2$ increase ($d[CO_2]/dt$) can be categorized as follows: $d[CO_2]/dt$ (Asthma) > $d[CO_2]/dt$ (Normal awake breathing) > $d[CO_2]/dt$ (Normal sleep breathing). Within this categorization, a further distinction in general can be made between the different types of asthma symptoms as follows: $d[CO_2]/dt$ (Shortness of breath) > $d[CO_2]/dt$ (Coughing) > $d[CO_2]/dt$ (Wheezing). Additionally or alternatively, the monitoring system 10 may be configured to count the number of fluctuations in the monitored $CO_2$ concentration over a defined period of time, e.g. to establish a pattern in the $CO_2$ signal obtained from the sensor 21, which pattern may be evaluated to determine the type of asthma symptom. Typically, over a defined time period, the measured number N fluctuations in the monitored $CO_2$ concentration corresponds to the various asthma symptoms as follows: N (Shortness of breath) > N (Coughing) > N (Wheezing). As will be understood from the foregoing, the various $d[CO_2]/dt$ thresholds or count (N) thresholds for establishing the particular types of asthma symptoms may be obtained through calibration or machine learning.

Hence, based on the foregoing it will be readily understood that by evaluating the rate of increase of monitored $CO_2$ levels, e.g. by comparing the monitored rate of increase against appropriately defined thresholds, a distinction can be made between whether the increase in $CO_2$ levels are caused by normal breathing or are caused by specific asthma symptoms, e.g. shortness of breath, coughing or wheezing, with each of the symptoms being identifiable due to the characteristic rates of $CO_2$ increases associated with each of these symptoms.

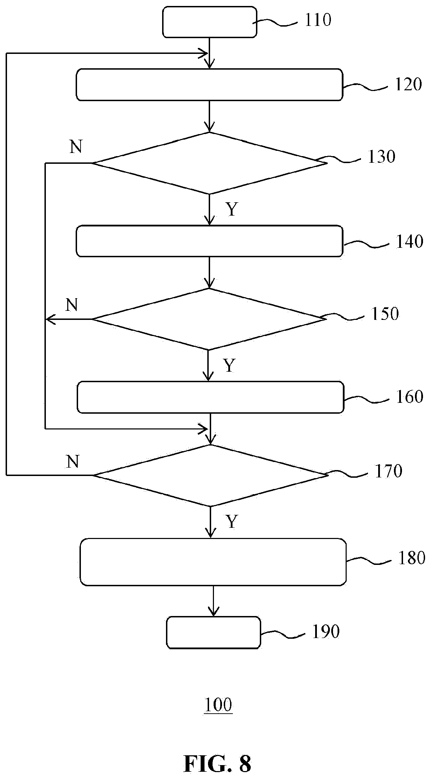

In an embodiment, the sleep monitoring system 10 is adapted to implement a method of detecting asthma symptoms during the sleep of a subject within the context of determining the sleep efficiency of the monitored subject. A flowchart of an example embodiment of such a method 100 is depicted in FIG. 8. The method 100 starts in operation 110, e.g. by switching on the sleep monitoring system 10, after which the processor 31 monitors the sensor data provided by the $CO_2$ sensor 21 and one or more further sensors 23 if present to determine whether the monitored subject is awake or asleep, which is checked in operation 130. Such monitoring of the sensor data by the processor 31 may be achieved in any suitable manner, e.g. periodically with any suitable periodicity such as at least once every 10 seconds as previously mentioned. The determination of whether the subject is awake or asleep and the determination of the sleep efficiency and related parameters such as sleep onset may be determined as explained in more detail above.

If it is determined in operation 130 that the subject is not yet asleep, the method 100 proceeds to operation 170 in which it is checked whether the monitoring period has lapsed. If this is not yet the case, the method 100 reverts back to previously explained operation 120. Otherwise, the method 100 proceeds to operation 180 in which the overall sleep efficiency of the monitored subject maybe calculated as explained in more detail above before terminating in 190. On the other hand, if it is determined in operation 130 that the subject is asleep, the method 100 proceeds to operation 140 in which the sleep level, e.g. normal sleep phases such as light sleep or deep sleep, or asthma affected sleep phases including sleep phases affected by coughing, shortness of breath or wheezing, is determined. Such monitoring may be continued as symbolically represented by operation 150 until the monitored subject wakes up or until the monitoring period during which the sleep monitoring system monitors the subject has completed.

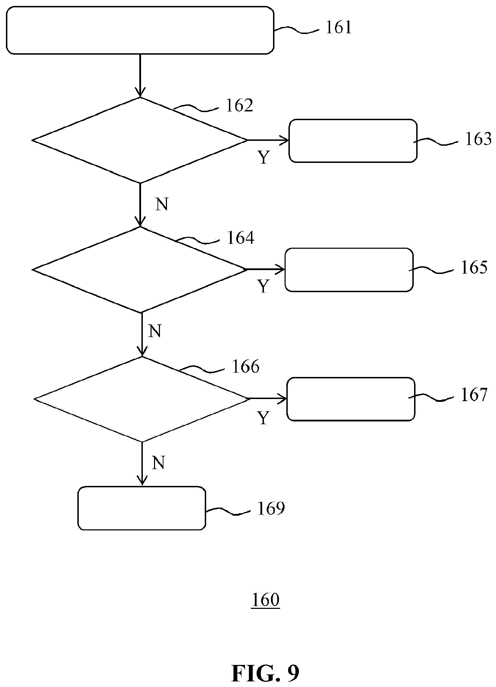

If it is determined in operation 150 that the subject has woken up, which for example may be evident from the monitored $CO_2$ data and/or from data provided by one or more of the further sensors 23 as previously explained, the method 100 optionally may comprise further operation 160 in which the cause for the subject waking up is determined. This is further explained with the aid of the flowchart in FIG. 9. Specifically, the processor 31 may evaluate a rate of increase in monitored $CO_2$ levels during a time interval immediately preceding the waking up event of the monitored subject in operation 161, as the rate of increase of the $CO_2$ levels during this time interval are indicative of the reason why the person has woken up, i.e. whether or not this was caused by a particular asthma symptom. For example, the processor 31 may compare the rate of increase in monitored $CO_2$ levels in this time interval against a first threshold indicative of shortness of breath in operation 162 such that if the monitored $CO_2$ levels at least meet this first threshold, the processor terminates in 163, which defines the reason for waking up as being caused by shortness of breath.

However, if the rate of increase in monitored $CO_2$ levels lie below this first threshold, the processor 31 may compare the rate of increase in monitored $CO_2$ levels in this time interval against a second threshold indicative of coughing in operation 164 such that if the monitored $CO_2$ levels at least meet this second threshold, the processor terminates in 165, which defines the reason for waking up as being caused by coughing.

If the rate of increase in monitored $CO_2$ levels also lie below this second threshold, the processor 31 may compare the rate of increase in monitored $CO_2$ levels in this time interval against a third threshold indicative of wheezing in operation 166 such that if the monitored $CO_2$ levels at least meet this third threshold, the processor terminates in 167, which defines the reason for waking up as being caused by wheezing. If the rate of increase in monitored $CO_2$ levels also lie below this third threshold, the processor 31 determines that the waking up of the monitored subject was not caused by the onset of asthma symptoms and consequently terminates in 169, indicating 'normal' waking up, i.e. waking up for non-asthma related reasons of the monitored subject.

In this manner, the sleep monitoring system 100 can build up a record of the number of asthma events and their duration during the sleep of the monitored subject, from which the deterioration of the sleep efficiency of the monitored subject can be quantified in any suitable manner. Such information for example may be used by the subject or a medical consultant of the subject to evaluate the efficiency of an actual treatment regime and to adjust the treatment regime accordingly, e.g. increase the dosage and/or administration frequency of already used asthma medication, or to supplement or replace the already used asthma medication with new medication to suppress the onset of asthma symptoms during the sleep of the monitored subject.

In this respect, the determination of the sleep efficiency may benefit from the determination of the number of times the monitored subject wakes up during the monitoring period as well as the reasons for the monitored subject waking up as previously explained with the aid of FIG. 9 and its detailed description, e.g. by counting the number of occasions the monitored subject has woken up because of nocturnal asthma symptoms. For example, the overall sleep efficiency may be expressed as the ratio between the total time asleep over the total monitoring period, e.g. the period in which the subject was in bed and trying to sleep. A decreased sleep efficiency figure may also be derived based on the difference between the sleep efficiency without asthma disturbance and the overall sleep efficiency, each of which may be derived from the monitored data in a straightforward manner using the teachings of the present invention as described in this application.

As previously explained, the monitoring results may be made available either to a remote monitor or on the monitoring result reporting device 35. Any useful results may be presented in any suitable manner. Non-limiting examples of useful results include sleep efficiency, wake time, sleep disturbance time caused by asthma, a quantification in terms of ranking of the sleep quality, e.g. on a scale of 1-5, with an increasing score indicative of higher sleep quality, and so on. In addition, such monitoring results may be supplemented by proposed suggestions to improve the sleep of the monitored subject, such as suggestions in adjusting asthma medication (for which the system may suggest that the subject discusses such adjustments with a medical professional where appropriate), the installation or use of devices that can reduce concentrations of airborne triggers of asthma, e.g. air purification devices or the like, and so on.

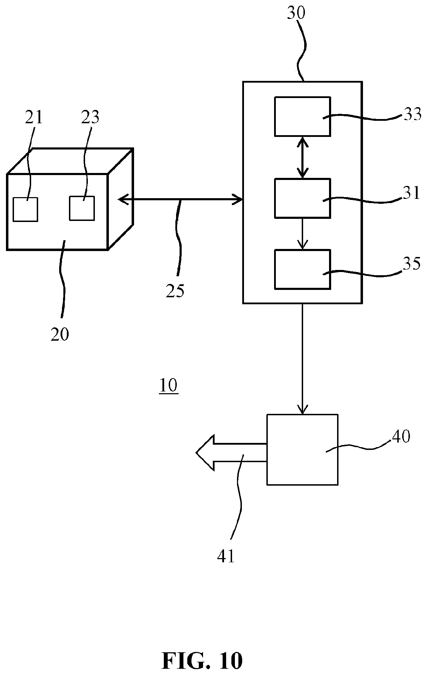

In an embodiment schematically depicted in FIG. 10, the sleep monitoring system 10 further comprises an asthma medication release device 40 responsive to the processor 31. For example, the asthma medication release device 40 may be an aerosol releasing device or the like adapted to release a flow 41 of the asthma medication in the direction of the mouth of the monitored subject, such that the processor 31 when detecting the onset of an asthma symptom in the monitored $CO_2$ data as previously explained can control the asthma medication release device 40 to release the asthma medication towards the monitored subject in order to automatically the asthma symptoms of the monitored subject.

In an embodiment, the dosage of the asthma medication released by the asthma medication release device 40 may be based on the particular asthma symptom detected with the processor 31. For example, the released dosage may be higher when the monitored subject suffers from shortness of breath or is wheezing compared to when the monitored subject is coughing as in the latter scenario the breathing of the monitored subject typically is less shallow, such that a smaller dosage of the asthma medication may suffice in effectively treating the asthma.

In an embodiment, the processor 31 may be responsive to a further sensor 23 adapted to detect a breathing cycle of the monitored subject, e.g. a motion sensor, camera or the like from which it may be determined whether the monitored subject is breathing in or out, such that the processor 31 can trigger the release of the asthma medication by the asthma medication release device 40 at the appropriate point in time such that the asthma medication reaches the monitored subject at the start of the subject breathing in air during the breathing cycle in order to maximize the fraction of the delivered asthma medication being inhaled by the monitored subject.

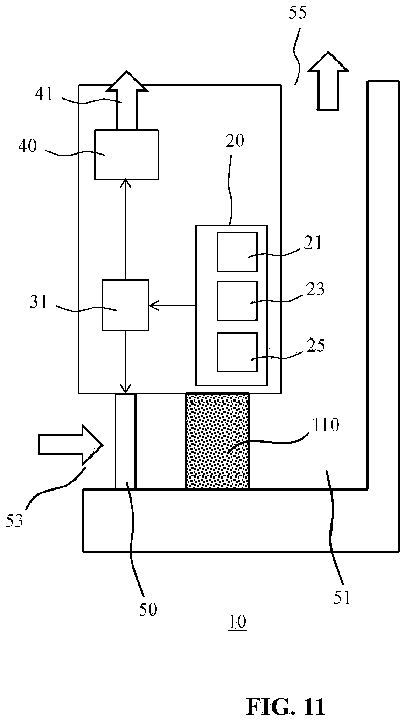

FIG. 11 schematically depicts an example embodiment of a sleep monitoring system 10 embodied by an air purification apparatus having an air inlet 53, an air outlet 55 and an air purification path 51, e.g. a fluid conduit, extending between the air inlet 53 and the air outlet 55. A fan 50 may be located in the air purification path 51 to control the flow rate of air through the air purification path 51. The fan 50 may be controlled by the processor 31 integral to the air purification apparatus. The processor 31 may be adapted to control a speed of the fan 50 in response to the detection of a particular sleep phase of the subject to be monitored, for example to maintain a desirable atmosphere within the space in which the subject is located. In this embodiment, the sensor device 20 is integral to the air purification apparatus and in addition to the previously described $CO_2$ sensor 21 and the one or more further sensors 23 may further comprise one or more additional sensors 25 for monitoring a pollution level in the air entering the air purification apparatus through the air inlet 53. The processor 31 may be further adapted to regulate the fan speed of the fan 50 in response to sensor data provided by the one or more additional sensors 25 to ensure that the air quality in the confined space in which the air purification apparatus is placed is appropriately regulated. As such sensor-based fan speed regulation is well-known per se, this is not further explained for the sake of brevity only.

The air purification apparatus further comprises one or more pollutant removal structures 110, e.g. filters or the like, as is well-known per se. This will therefore not be further explained for the sake of brevity only. The air purification apparatus may further comprise an asthma medication delivery device 44 delivering a flow 41 including an asthma medication as previously explained. It will be immediately understood by the skilled person that although such an integral sleep monitoring system 10 is explained in more detail for an air purification apparatus, it is equally feasible to implement such an integral sleep monitoring system 10 in similar air treatment apparatuses such as air conditioners, air humidifiers, respirator devices, and so on.

At this point, it is noted that embodiments of the sleep monitoring system 10 may also be used in a confined space in which multiple subjects are sleeping. For example, in such a scenario multiple sleep monitoring systems with ($CO_2$) sensing capability local to a particular subject may be deployed. Alternatively, a single sleep monitoring system 10 comprising multiple sensors which may be deployed local to a particular subject to be monitored may be contemplated. Although embodiments of the present invention are described in the context of $CO_2$ monitoring, it should be understood that alternative embodiments in which another gas affected by ventilation (breathing), e.g. $O_2$, may be monitored, e.g. to support or replace $CO_2$ monitoring data. Other sensors that may be advantageously deployed to monitor sleep disorders and the onset of asthma symptoms include volatile organic compound (VOC) sensors, which for example may be deployed as the further sensors 23 to supplement the data obtained with the $CO_2$ sensor 21.

Aspects of the present invention may be embodied as sleep monitoring system 10 and a method 100 for monitoring the sleep of a subject. Aspects of the present invention may take the form of a computer program product embodied in one or more computer-readable medium(s) having computer readable program code embodied thereon. The code typically embodies computer-readable program instructions for, when executed on a processor 31 of such a sleep monitoring system 10, implementing the sleep monitoring method 100.

Any combination of one or more computer readable medium(s) maybe utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. Such a system, apparatus or device may be accessible over any suitable network connection; for instance, the system, apparatus or device may be accessible over a network for retrieval of the computer readable program code over the network. Such a network may for instance be the Internet, a mobile communications network or the like. More specific examples (a non-exhaustive list) of the computer readable storage medium may include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present application, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out the methods of the present invention by execution on the processor 31 may be written in any combination of one or more programming languages, including an object oriented programming language such as Java, Smalltalk, C++ or the like and conventional procedural programming languages, such as the "C" programming language or similar programming languages. The program code may execute entirely on the processor 31 as a standalone software package, e.g. an app, or may be executed partly on the processor 31 and partly on a remote server. In the latter scenario, the remote server may be connected to the sleep monitoring system 10 through any type of network, including a local area network (LAN) or a wide area network (WAN), or the connection maybe made to an external computer, e.g. through the Internet using an Internet Service Provider.

Aspects of the present invention are described above with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions to be executed in whole or in part on the processor 31 of the sleep monitoring system 10, such that the instructions create means for implementing the functions/acts specified in the flowchart and/or block diagram block or blocks. These computer program instructions may also be stored in a computer-readable medium that can direct the sleep monitoring system 10 to function in a particular manner.

The computer program instructions may be loaded onto the processor 31 to cause a series of operational steps to be performed on the processor 31, to produce a computer-implemented process such that the instructions which execute on the processor 31 provide processes for implementing the functions/acts specified in the flowchart and/or block diagram block or blocks. The computer program product may form part of the sleep monitoring system 10, e.g. may be installed on the sleep monitoring system 10.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A sleep monitoring system (10) for detecting asthma symptoms during the sleep of a subject, the system comprising a $CO_2$ sensor (21) and a processor (31) communicatively coupled to the $CO_2$ sensor, wherein the processor is adapted to:

   monitor a change in a $CO_2$ concentration in a space in which the subject is asleep from sensor data produced by the $CO_2$ sensor over a monitoring period;
   compare the monitored change of the $CO_2$ concentration in said space against a benchmark for said subject; and
   identify an asthma symptom exhibited by said subject if the monitored change of the $CO_2$ concentration in said space exceeds the benchmark for said subject for at least part of said monitoring period.

2. The sleep monitoring system (10) of claim 1, further comprising a monitoring result reporting device (35) communicatively coupled to the processor (31) and adapted to generate a report of said monitoring period, said report including an indication of identified asthma symptoms.

**3.** The sleep monitoring system (10) of claim 1 or 2, wherein the processor (31) further is adapted to sample the sensor data at a defined sampling frequency of at least 0.1 Hz.

**4.** The sleep monitoring system (10) of claim 3, wherein the processor (31) is adapted to identify an asthma symptom in the form of a cough of the subject if a monitored change in said $CO_2$ concentration between at least two contiguous sensor data samples exceeds the benchmark by at least a defined amount.

**5.** The sleep monitoring system (10) of any of claims 1-4, further comprising a counter adapted to count the number of identified asthma symptoms during the monitoring period.

**6.** The sleep monitoring system (10) of any of claims 1-5, wherein the system is operable in a calibration mode in which the benchmark for the subject is obtained.

**7.** The sleep monitoring system (10) of any of claims 1-6, wherein the monitoring period is defined by a first time period initiated by a first indication that the subject is attempting to sleep and terminated by a second indication that the subject is getting up; and wherein the processor (31) is adapted to determine a sleep efficiency of the subject during the monitoring period by:

identifying an awake time period of the subject when a rate of increase in the monitored $CO_2$ concentration is greater than a first threshold; identifying a light sleep time period of the subject when a rate of increase in the monitored $CO_2$ concentration is between the first threshold and a second threshold; and identifying a deep sleep phase of the subject when a rate of increase in the monitored $CO_2$ concentration is below the second threshold.

**8.** The sleep monitoring system (10) of claim 7, wherein the processor (31) is further adapted to determine a cause for the subject being awake by evaluating a rate of increase in the monitored CO2 concentration during a time period immediately preceding the awake time period.

**9.** The sleep monitoring system (10) of claim 8, wherein said causes including normal waking up and asthma symptoms including coughing, shortness of breath and wheezing.

**10.** The sleep monitoring system (10) of any of claims 1-9, further comprising at least one further sensor (23) communicatively coupled to the processor (31), wherein the processor is adapted to identify the asthma symptoms based on respective sensor data from the $CO_2$ sensor (21) and the at least one further sensor.

**11.** The sleep monitoring system (10) of any of claims 1-10, further comprising an asthma medication release device (40) responsive to the processor (31).

**12.** The sleep monitoring system (10) of any of claims 1-11, wherein the sleep monitoring system is at least partially comprised by an air conditioning apparatus, an air purification apparatus, a respirator apparatus or a humidification apparatus, and/or wherein the sleep monitoring system comprises a first device (20) comprising the $CO_2$ sensor (21) and a second device (30) comprising the processor (31), the first device and the second device each comprising a wireless communication module for establishing a wireless communication link between the first device and the second device, wherein the second device is a wearable device or a mobile communication device.

**13.** A method (100) of detecting asthma symptoms during the sleep of a subject, the method comprising:

periodically receiving (110) sensor data indicative of a $CO_2$ concentration in a space in which the subject is asleep during a monitoring period; monitoring (120) the CO2 concentration over said monitoring period; comparing (160) a change in the monitored $CO_2$ concentration in said space against a benchmark for said subject; and identifying (162, 164, 166) an asthma symptom exhibited by said subject if the monitored change of the $CO_2$ concentration in said space exceeds the benchmark for said subject for at least part of said monitoring period.

**14.** The method (100) of claim 13, further comprising:

determining a sleep efficiency of the subject during the monitoring period by:

identifying (150) an awake time period of the subject when a rate of increase in the monitored $CO_2$ concentration is greater than a first threshold; identifying (140) a light sleep time period of the subject when a rate of increase in the monitored $CO_2$ concentration is between the first threshold and a second threshold; and identifying (140) a deep sleep phase of the subject when a rate of increase in the monitored $CO_2$ concentration is below the second threshold;

the determining of said sleep efficiency further comprising determining (160) a cause for the subject being awake by evaluating a rate of increase in the monitored $CO_2$ concentration during a time period immediately preceding the awake time period from a set of causes including normal waking up and asthma symptoms including coughing, shortness of breath and wheezing.

**15.** A computer program product comprising a computer readable storage medium having computer readable program instructions embodied therewith for, when executed on a processor (31) of a sleep monitoring system (10) of any of claims 1-12, cause the processor to implement the method (100) of claim 13 or 14.

10

**FIG. 1**

**FIG. 2**

**FIG. 3**

**FIG. 4**

$$y = 1.6339x + 1430.9$$
$$R^2 = 0.974$$

**FIG. 5**

**FIG. 6**

**FIG. 7**

FIG. 8

FIG. 9

**FIG. 10**

**FIG. 11**

Europäisches
Patentamt

European
Patent Office

Office européen
des brevets

# EUROPEAN SEARCH REPORT

**Application Number**

EP 17 17 0699

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/228037 A1 (BUBIS YACOV [IL] ET AL) 11 August 2016 (2016-08-11) | 1-12 | INV. G06F19/00 |
| Y | * par. 4, 7, 11, 13, 15, 64, 70; claim 11 * | 12 | |
| Y | US 2016/174907 A1 (COLMAN JOSHUA LEWIS [IL] ET AL) 23 June 2016 (2016-06-23) | 12 | |
| A | * par. 128, 146 * | 1-11, 13-15 | |
| A | Anonymous: "REM, Light, Deep: How Much of Each Stage of Sleep Are You Getting? - Fitbit Blog", 15 March 2017 (2017-03-15), XP055422904, Retrieved from the Internet: URL:https://web.archive.org/web/2017031506 2944/https://blog.fitbit.com/sleep-stages-explained/ [retrieved on 2017-11-08] * page 3 rd and 4th par * | 1-15 | |
| A | DIMITRIOS KIKIDIS ET AL: "The Digital Asthma Patient: The History and Future of Inhaler Based Health Monitoring Devices", JOURNAL OF AEROSOL MEDICINE AND PULMONARY DRUG DELIVERY, vol. 29, no. 3, 1 June 2016 (2016-06-01), pages 219-232, XP055422814, US ISSN: 1941-2711, DOI: 10.1089/jamp.2015.1267 * page 223 3rd and 4th par * | 1-15 | TECHNICAL FIELDS SEARCHED (IPC) G06F |

-/--

The present search report has been drawn up for all claims

| Place of search | Date of completion of the search | Examiner |
|---|---|---|
| Munich | 10 November 2017 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 17 17 0699

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Malarvili Balakrishnan ET AL: "A review of capnography in asthma: A new approach on assessment of capnogram", , 18 January 2013 (2013-01-18), XP055422798, Retrieved from the Internet: URL:https://www.researchgate.net/profile/Mohsen_Kazemi2/publication/259079197_A_review_of_capnography_in_asthma_A_new_approach_on_assessment_of_capnogram/links/02e7e529e8fa4ab919000000/A-review-of-capnography-in-asthma-A-new-approach-on-assessment-of-capnogram.pdf [retrieved on 2017-11-08] * page 555 last par * ----- | 1-15 | |

TECHNICAL FIELDS
SEARCHED      (IPC)

The present search report has been drawn up for all claims

| Place of search | Date of completion of the search | Examiner |
|---|---|---|
| Munich | 10 November 2017 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 17 0699

10-11-2017

| Patent document cited in search report | Publication date | Patent family member(s) | Publication date |
|---|---|---|---|
| US 2016228037 A1 | 11-08-2016 | US 2016228037 A1<br>WO 2016128958 A1 | 11-08-2016<br>18-08-2016 |
| US 2016174907 A1 | 23-06-2016 | EP 2303376 A2<br>EP 2374493 A2<br>JP 2011522583 A<br>US 2011098592 A1<br>US 2013289364 A1<br>US 2014155775 A1<br>US 2014155776 A1<br>US 2016174907 A1<br>US 2017296095 A1<br>WO 2009144731 A2 | 06-04-2011<br>12-10-2011<br>04-08-2011<br>28-04-2011<br>31-10-2013<br>05-06-2014<br>05-06-2014<br>23-06-2016<br>19-10-2017<br>03-12-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160249838 A1